# EUROPEAN PATENT APPLICATION

(11) **EP 4 210 071 A1**
(43) Date of publication of application: **12.07.2023**
(21) Application number: 22150530.8
(22) Date of filing: 07.01.2022
(51) Int. Cl.: G16H 50/20, G16H 50/50

(54) **MACHINE LEARNING ARCHITECTURE FOR MEDICAL CODING**

(71) Applicant: Bayer AG, 51373 Leverkusen (DE)
(72) Inventor: BERNS, Christoph, 60388 Frankfurt am Main (DE)
(74) Representative: BIP Patents

(57) **Abstract**

The present disclosure relates to the technical area of medical coding. Aspects of the present disclosure relate to a computer system and a computer readable medium with instructions to a processor for supporting medical coding activities of one or more users.

## Description

### FIELD

The present disclosure relates to the technical area of medical coding. Aspects of the present disclosure relate to a computer system and a computer readable medium with instructions to a processor for supporting medical coding activities of one or more users.

### BACKGROUND

Medical coding is the transformation of medical information, such as information related to healthcare diagnosis, procedures, medical services, and/or equipment, into universal medical alphanumeric codes.

Medical coding means taking free text entered into, e.g., electronic case report files, and mapping it to one or more entries in a medical dictionary.

The main objective of performing medical coding is to have medical terms interpreted uniformly and in a standardized format.

Uniquely identifying medical concepts in healthcare data is a key task in many processes in healthcare domains. Coding adverse event citations to the Medical Dictionary for Regulatory Activities (MedDRA), coding drug citations to the WHO Drug Dictionary (WHO-DD), coding medical condition citations to the International Classification of Diseases and Related Health Problems (ICD) are a few examples.

Traditionally, medical coding tasks are carried out by highly trained experts in the healthcare domain.

This mapping task is a challenging cognitive task for human practitioners even with pharma domain knowledge. This is due to the large number of possible codes and fine-grained semantic differences between the codes.

More recently, solutions have been developed to automate these complex coding tasks. For example, US20190340487A1 and US20200273573 disclose machine learning architectures for performing medical coding.

In the area of medical coding, changes occur frequently. For example, there are constant updates in medical dictionaries. This means that machine learning models used for automatic coding also need to be kept constantly up to date.

This and other problems are addressed in this disclosure.

### SUMMARY

The present disclosure provides means for supporting users in medical coding activities.

In a first aspect, the present disclosure provides a machine learning architecture for medical coding comprising:
- a serving unit, wherein the serving unit is configured to receive a reported term, to output a classification result and to receive feedback from a user,
- a classification model, wherein the classification model is configured to assign one or more medical codes to the reported term and to provide the classification result, the classification result comprising the one or more assigned codes and optionally a probability value, the probability value indicating how likely it is that the assigned code has been correctly assigned and/or that the assignment is complete,
- a data ingestion unit, wherein the data ingestion unit is configured to receive training data for training and/or re-training and/or further training of the classification model,
- a storage unit, wherein the storage unit is configured to store and provide ingested and/or processed data, model artifacts, and/or parameters,
- a transformation unit, wherein the transformation unit is configured to pre-process the training data and/or to compute model performance KPIs and/or data quality KPIs,
- a training unit, wherein the training unit is configured to train, re-train and/or further train the classification model on the basis of pre-processed training data,
- a monitoring and orchestration unit, wherein the monitoring and orchestration unit is configured to monitor the architecture and/or the classification model performance, to process data exchange and/or to reflect dependencies between units and/or to schedule (and reflect dependencies between) tasks of units.

In another aspect, the present disclosure provides a non-transitory computer readable medium having stored thereon software instructions that, when executed by one or more processors of one or more computers, cause the computer(s) to execute the following steps:
- receiving a plurality of reported terms,
- for each received reported term:
   ∘ determining one or more medical codes on the basis of the reported term by means of a trained classification model, the classification model being trained on the basis of a medical dictionary to assign one or more medical codes to a reported term,
   ∘ outputting the one or more medical codes,
   ∘ receiving user feedback, the user feedback indicating whether the one or more medical codes are correct and/or complete
- monitoring the number and/or the type of incorrect and/or incomplete medical codes on the basis of the user feedback,
- initiating further training and/or re-training in case the number and/or percentage of incorrect and/or incomplete medical codes and/or incorrect and/or incomplete types of medical codes exceeds a pre-defined threshold, and/or in case an update of the medical dictionary is available.

Preferred embodiments of the present disclosure can be found in the dependent patent claims, the present description, and in the drawings.

### DETAILED DESCRIPTION

The different aspects of the present disclosure will be more particularly elucidated below without distinguishing between the different subject matter of the patent claims (machine learning architecture, computer-readable storage medium). On the contrary, the following elucidations are intended to apply analogously to all subject matter of the patent claims, irrespective of in which context (machine learning architecture, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations will be described more fully hereinafter with reference to the accompanying drawings, in which some, but not all implementations are shown. Indeed, various implementations may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for supporting users in medical coding activities. These means comprise a machine learning architecture for medical coding.

Such an architecture can be a single computer or a computer system consisting of several computers. The term "architecture" is meant to indicate that there are different units that interact with each other. The units may be real-world devices and/or functionalities provided by one computer (monolithic architecture) or more computers (microservice architecture) and/or peripherals. Further, the units can be realized in terms of services provided by a cloud computing provider.

A "computer" is a machine that can be programmed to carry out sequences of arithmetic and/or logical operations automatically. Usually, computers can perform generic sets of operations known as programs. These programs enable computers to perform a wide range of tasks. Such a computer usually comprises one or more processors for carrying out the arithmetic and/or logical operations, and optionally peripherals. In computer technology, "peripherals" refer to all devices which are connected to the computer and serve for the control of the computer and/or as input and output devices. Examples thereof are monitor (screen), printer, scanner, mouse, keyboard, hard drive, camera, microphone, loudspeaker, etc. Internal ports and expansion cards are, too, considered to be peripherals in computer technology.

The machine learning architecture according to the present disclosure comprises:
- a classification model,
- a serving unit,
- a data ingestion unit,
- a storage unit,
- a transformation unit,
- a training unit, and
- a monitoring and orchestration unit.

The classification model is configured to receive a reported term and provide a classification result on the basis of the reported term.

A "reported term" is a textual description of a medical event. Often the medical event is described in a natural language. Examples of medical events are healthcare diagnosis, procedures, medical services, or equipment. A reported term may identify a drug, an effect, and/or information related to the drug and/or the effect. A reported term may be an adverse event term.

The classification model is configured to assign one or more medical codes to a reported term. The assignment is usually done on the basis of a medical dictionary, such as the Medical Dictionary for Regulatory Activities (MedDRA), the WHO Drug Dictionary (WHO-DD), the dictionary for International Classification of Diseases and Related Health Problems (ICD) and/or any other medical dictionary.

The classification model is configured to provide a classification result, the classification result comprising the one or more assigned medical codes and optionally a probability value, the probability value indicating how likely it is that the one or more assigned codes have been correctly assigned and/or that the assignment is complete.

The classification model is a machine learning model. Such a "machine learning model", as used herein, may be understood as a computer implemented data processing architecture. The machine learning model can receive input data (such as a reported term) and provide output data (such as one or more medical codes) based on that input data and the machine learning model, in particular parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

The training data can comprise a multitude of reference reported terms that have been assigned (e.g., by medical experts) to one or more medical codes, and, for each reference reported term, the respective one or more medical codes assigned to it.

The term "multitude" as it is used herein means an integer greater than 10, usually greater than 100, preferably greater than 1000.

In the training process, the reference reported terms of the training data are inputted into the machine learning model and the machine learning model generates an output. The output is compared with the (known) target (the one or more medical codes assigned to the reference medical codes). Parameters of the machine learning model are modified in order to reduce the deviations between the output and the (known) target to a (defined) minimum.

In general, a loss function can be used for training to evaluate the machine learning model. For example, a loss function can include a metric of comparison of the output and the target. The loss function may be chosen in such a way that it rewards a wanted relation between output and target and/or penalizes an unwanted relation between an output and a target. Such a relation can be, e.g., a similarity, or a dissimilarity, or another relation. A loss function can be used to calculate a loss value for a given pair of output and target. The aim of the training process can be to modify (adjust) parameters of the machine learning model in order to reduce the loss value to a (defined) minimum.

The classification model according to the present disclosure can be, e.g., the neural network disclosed in US20190340487A1 and/or the machine learning model disclosed in US20200273573 and/or any other comparable (similar) classification model.

In addition to a classification model, the machine learning architecture of the present disclosure comprises means to evaluate the quality (e.g., the predictive accuracy) of the model and/or the quality of the training data (e.g., data drift detection) and to initiate training procedures when an update of the model is required.

The machine learning architecture comprises a serving unit. The serving unit can serve as a communication interface to one or more users. Such a user is usually a human person, for example an expert in the field of medical coding or another computer system which supports the process of medical coding.

The serving unit is configured to receive reported terms and to output classification results and to receive feedback from the one or more users.

A reported term can be inputted into the serving user, e.g., by a user via a keyboard and/or a microphone. Usually reported terms are stored in a data storage, and a user can select one or more reported terms to be assigned one or more medical codes via the serving unit, e.g., using a mouse, a touch-sensitive surface, a keyboard, a microphone and/or any other suitable input device for selecting items stored in a data storage. The serving unit can be configured to feed the one or more reported terms to the classification unit and receive, as an output from the classification unit, the classification result. The serving unit can be configured to output the classification result, e.g., to display it on a monitor, print it on a printer and/or store it into a data storage. The serving unit may also be configured to transmit the classification result to another computer.

The classification result is presented to a user. Optionally, besides the classification result, the reported term on the basis of which the classification result was generated is presented to the user as well. Optionally, a probability value is presented to the user as well, the probability value indicating how likely it is that the classification result is correct, i.e., that the assigned code has been correctly assigned and/or that the assignment is complete.

Preferably, a feedback is requested from the user. The feedback may indicate whether the medical code(s) assigned to a reported term are correct or incorrect and/or complete or incomplete. For example, for each medical code assigned to a reported term, the user is requested to indicate whether the medical code is correct or incorrect. The user can be requested to provide feedback whether the assigned medical code(s) is(are) complete or whether one or more additional medical codes need to be assigned to a reported term. The user can provide feedback, for example, by clicking a virtual box. In the event that a user provides the feedback that an assigned medical code is incorrect (or incomplete), the user may be requested to input the right (or complete or missing) medical code into the computer system via the serving unit.

Similarly, the classification result may include a plurality of possible medical codes, e.g., two or three or four or five or a higher number of possible medical codes. Preferably, a probability value is provided for each possible medical code, wherein the probability value indicates how likely it is that the respective medical code is *a* correct medical code (in case of more than one applicable medical codes) or *the* correct medical code (in case of only one applicable medical code).

Preferably, only those possible medical codes are outputted (e.g., displayed) whose probability is above a predefined threshold. This threshold may be, for example, 50% or 60% or 70% or 80% or 90% or 95% or another percentage or probability value.

Preferably the possible medical codes are outputted in the order of probability that they are correct, preferably the possible medical code with the highest probability is outputted/displayed first and/or at the top. A user may be prompted to select the medical code / medical codes that is / are correct. The medical codes that are not selected can be classified as incorrect. In this case, the user's feedback comprises of a number of correct and/or incorrect codes.

Preferably, the feedback is stored as user feedback data in a data storage (e.g., of the storage unit). Preferably the user feedback data are used (e.g., at a later stage) for re-training purposes (see below).

The machine learning architecture comprises a data ingestion unit. The data ingestion unit is configured to receive training data for training and/or re-training and/or further training of the classification model. The data ingestion unit can, e.g., be configured to receive an updated dictionary and/or user feedback data and/or (other) new training data. Instead of and/or in addition to training data, the data ingestion unit may also be configured to receive information about (newly) available training data and the locations where the data can be retrieved. The data ingestion unit may be configured to retrieve newly available training data once they are available and/or if the monitoring and orchestration unit triggers the retrieval and/or at defined times and/or at defined intervals and/or when defined events occur.

The training data received by the data ingestion unit is usually not data that can be used directly to train (or re-train or further train) the classification model. Instead, the training data must be pre-processed. The pre-processing of training data is performed by the transformation unit. During pre-processing, the training data are converted into a form in which they can be used by the learning algorithm to train the classification model. Usually, training pairs are generated, wherein each training pair usually comprises a reference reported term and one or more associated medical codes. The transformation unit can be configured to identify duplicates and/or other data quality issues in training data and to remove them.

For the result of pre-processing, the term "pre-processed training data" is used in this disclosure.

Newly available training data may be or comprise an updated dictionary. The transformation unit may be configured to compare the updated dictionary with the previous dictionary to identify new and/or changed entries. If there are new entries, the transformation unit may be configured to identify and/or generate training pairs for the new entries so that the classification model can learn the new entries. If the updated dictionary contains changed entries, the transformation unit may be configured to generate changed training pairs that take the changes into account.

Preferably, the ingestion unit and/or the transformation unit have/has access to user feedback data from one or more users to generate training pairs from the user feedback data for training the classification model. Whenever a user has provided feedback that a reported term has been incorrectly (or incompletely) assigned to one or more medical codes, a training pair comprising the reported term and the correct (complete) medical code entered and/or selected by the user can be created by the transformation unit and used for training.

The machine learning architecture of the present disclosure comprises a training unit. The training unit is configured to train, re-train and/or further train the classification model on the basis of pre-processed training data. The training unit usually contains the learning algorithm including one or more loss functions to control the training process.

The machine learning architecture comprises a storage unit. The storage unit is used to store and provide data, such as training data, pre-processed training data, user feedback data, one or more trained and/or untrained machine learning models including model parameters, reported terms, and/or one or more dictionaries. It is possible that there is more than one storage unit.

The machine learning architecture comprises a monitoring and orchestration unit. The monitoring and orchestration unit is configured to monitor the processes that take placed in the machine learning architecture and to log all relevant activities. For example, the monitoring and orchestration unit can monitor the model training both in terms of hardware consumption (e.g., GPUs/CPUs) and model performance (e.g., predictive accuracy). The monitoring and orchestration unit may be configured to monitor how often a user declares a classification performed by the classification model as correct and/or incorrect and/or as complete and/or as not complete.

The monitoring and orchestration unit may be configured to generate and output a notification to a user that re-training of the classification model may be necessary when a pre-defined threshold number (or percentage) of incorrect assignments is exceeded. The monitoring and orchestration unit may be configured to initiate re-training of the classification model when a pre-defined threshold number (or percentage) of incorrect assignments is exceeded.

The monitoring and orchestration unit may be configured to indicate the need for a re-training and/or to initiate re-training of the classification model if an incorrect medical code has been repeatedly assigned to a specific reported term.

The monitoring and orchestration unit may be configured to analyze probabilities of correctness of assignments and, in the event that the probability for one or more specific reported terms (types of reported terms) is below a defined threshold (e.g., below 50% or 40% or 30% or any other percentage), to indicate the need for further training of the classification model and/or to initiate the further training of the classification model. If a (type of) reported term is repeatedly assigned to one or more medical codes with a comparatively low probability, this may indicate that no or too few data representing the (type of) reported term were used during training. Further training may be required.

In a preferred embodiment, the machine learning architecture of the present disclosure is provided in the form of Infrastructure-as-Code. Infrastructure-as-Code (IaC) is a practice in information technology (IT) that codifies and manages underlying IT infrastructure as software. The purpose of IaC is to enable developers and/or operations teams to automatically manage, monitor and provision resources, rather than manually configure discrete hardware devices and operating systems. IaC is sometimes referred to as programmable or software-defined infrastructure. The concept of IaC is like programming scripts, which are used to automate IT processes or to implement business logic. However, scripts are primarily used to automate a series of static steps that are repeated numerous times. IaC uses higher-level or descriptive language to code more versatile and adaptive provisioning and deployment processes.

In a preferred embodiment, the machine learning architecture of the present disclosure comprises a configuration unit with which the machine learning architecture can be defined, configured, generated, and maintained in the form of IaC.

Aspects of the present disclosure are explained in more detail below with reference to the drawings, without intending to limit the disclosure to the features and combinations of features shown in the drawings.

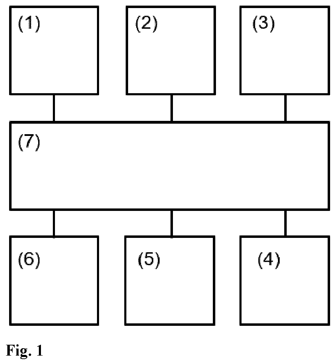

Fig. 1 shows schematically a preferred embodiment of the machine learning architecture of the present disclosure.

The machine learning architecture comprises a classification model (1), a serving unit (2), a data ingestion unit (3), a storage unit (4), a transformation unit (5), a training unit (6), and a monitoring and orchestration unit (7).

In Fig. 1, the machine learning architecture is shown as a centralized system, where the monitoring and orchestration unit (7) is connected to the other components and controls the data flow between them. Alternatively, the machine learning architecture can be designed as a decentralized system, where the individual components are connected to each other and exchange data. A mixed system, in which some or all components are connected to a central unit and some or all components are connected to each other, is also conceivable.

The classification model (1) is configured to generate, on the basis of a reported term, a classification result. The classification model (1) can be stored in a non-volatile data memory. The classification model (1) can be loaded into a working memory of a computer.

The serving unit (2) is configured to receive a reported term, to output a classification result and to receive feedback from a user. The serving unit (2) usually has one or more input devices and output devices. The serving unit (2) can access (directly or indirectly, e.g., via the monitoring and orchestration unit) the storage unit (4), e.g., in order to load a trained model into the working memory.

The data ingestion unit (3) is configured to receive training data for training and/or re-training and/or further training of the classification model (1).

The storage unit (4) is configured to store and provide data, such as training data and/or pre-processed training data and/or user feedback data, reported terms, one or more dictionaries, the classification model (1) including model parameters, and/or the like. The storage unit (4) can comprise one or more data storages. It is possible that there is more than one storage unit.

The transformation unit (5) is configured to pre-process the training data and to compute model performance and/or data quality KPIs (KPI: key performance indicator).

The training unit (6) is configured to train, re-train and/or further train the classification model (1) on the basis of pre-processed training data.

The monitoring and orchestration unit (7) is configured to monitor the machine learning architecture, to process data exchange between the different units and/or to schedule tasks of the different units.

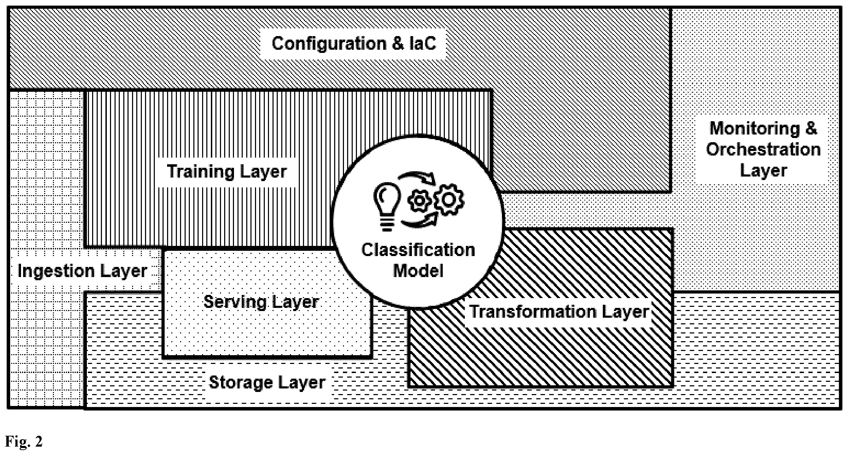

Fig. 2 shows the machine learning architecture of the present disclosure in the form of Infrastructure-as-Code. At the heart of the system is the classification model. The individual units of the machine learning architecture are referred to here as layers. The Serving Layer corresponds to the serving unit. The Ingestion Layer corresponds to the data ingestion unit. The Storage Layer corresponds to the storage unit. The Transformation Layer corresponds to the transformation unit. The Monitoring & Orchestration Layer corresponds to the monitoring and orchestration unit. There is an additional layer referred to as Configuration & IaC with which the machine learning architecture can be defined, configured, generated, and maintained.

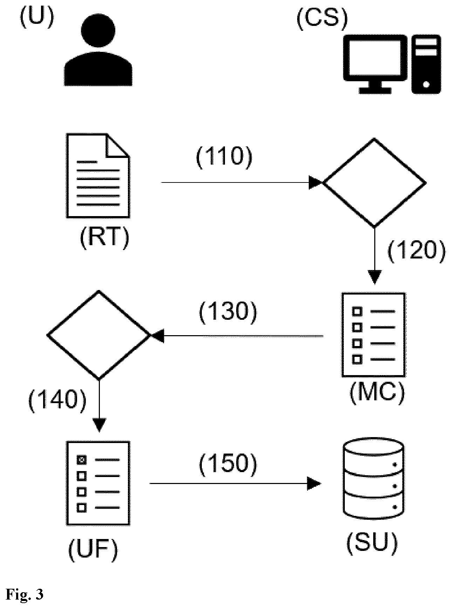

Fig. 3 shows a typical course of use of the machine learning architecture by a user. The machine learning architecture is represented by the computer system (CS). In a first step (110), the user (U) inputs into the computer system (CS) or selects via a user interface to the computer system (CS) a reported term (RT). In a second step (120), the computer system (CS) generates, on the basis of the reported term (RT), a list of possible medical codes (MC). In a third step (130), the computer system (CS) provides the list of possible medical codes (MC) to the user (U), and requests feedback from the user (U). In a fourth step (140), the user (U) provides feedback by marking those medical codes that apply to the reported term (RT). In a fifth step (150), the user feedback (UF) is provided to the computer system (CS) and stored in a data storage of the storage unit (SU).

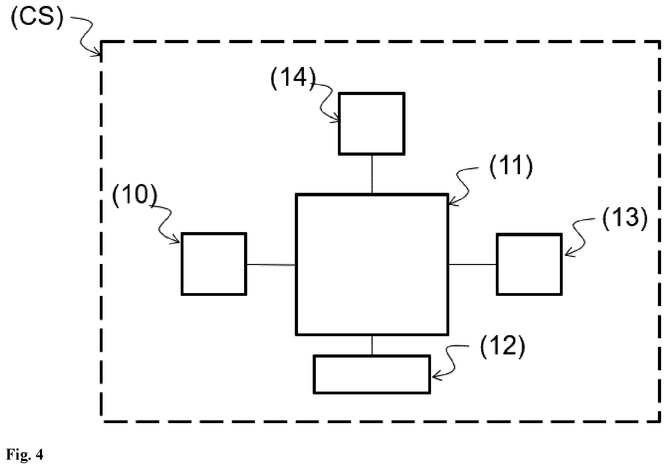

Fig. 4 shows schematically by way of example one embodiment of a computer.

Generally, a computer may comprise, include, or be embodied in one or more fixed or portable electronic devices.

The computer system (CS) comprises an input unit (10), a processing unit (11), a memory (12), an output unit (13), and communication interface(s) (14).

Data (e.g., training data, reported terms, user feedback data) can be entered into the computer system (CS) via the input unit (10). The input unit (10) can act as an interface to a user by accepting commands to control the computer system (CS). User input interface(s) may be wired or wireless and may be configured to receive information from a user into the computer system (CS), such as for processing, storage and/or display. Suitable examples of user input interfaces include a microphone, image or video capture device (e.g., a camera), keyboard or keypad, joystick, touch-sensitive surface (separate from or integrated into a touchscreen) and/or the like. In some examples, the user interfaces may include automatic identification and data capture (AIDC) technology for machine-readable information. This may include barcode, radio frequency identification (RFID), magnetic stripes, optical character recognition (OCR), integrated circuit card (ICC), and the like. The user interfaces may further include one or more interfaces for communicating with peripherals such as sensors, printers, and/or the like.

The processing unit (11) may be composed of one or more processors alone or in combination with one or more memories. The processing unit (11) is generally any piece of computer hardware that is capable of processing information such as, for example, data, computer programs and/or other suitable electronic information. The processing unit (11) is composed of a collection of electronic circuits some of which may be packaged as an integrated circuit or multiple interconnected integrated circuits (an integrated circuit at times more commonly referred to as a "chip"). The processing unit (11) may be configured to execute computer programs, which may be stored onboard the processing unit (11) or otherwise stored in the memory (12). The processing unit (11) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (11) may be implemented using a number of heterogeneous processor systems in which a main processor is present with one or more secondary processors on a single chip. It is even possible that the processing unit consists of independent sub-processing units. For example, the system of the present disclosure contains sub-processing units for model training (which are primarily GPU based) as well as sub-processing units for serving predictions (which are CPU based). As another illustrative example, the processing unit (11) may be a symmetric multi-processor system containing multiple processors of the same type. In yet another example, the processing unit (11) may be embodied as or otherwise include one or more ASICs, FPGAs or the like. Thus, although the processing unit (11) may be capable of executing a computer program to perform one or more functions, the processing unit (11) may be capable of performing one or more functions without the aid of a computer program. In either instance, the processing unit (11) may be appropriately programmed to perform functions or operations according to example implementations of the present disclosure.

The memory (12) is generally any piece of computer hardware that is capable of storing information such as, for example, data, computer programs (computer-readable program code) and/or other suitable information either on a temporary basis and/or a permanent basis. The memory (12) may include volatile and/or non-volatile memory and may be fixed or removable. Examples of suitable memory include random access memory (RAM), read-only memory (ROM), a hard drive, a flash memory, a thumb drive, a removable computer diskette, an optical disk, a magnetic tape, or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information and is distinguishable from computer-readable transmission media such as electronic transitory signals capable of carrying information from one location to another. Computer-readable medium as described herein may generally refer to a computer-readable storage medium or computer-readable transmission medium.

Information and/or data can be output from the computer system (CS) via the output unit (13). The output unit (13) may be or comprise one or more component(s) that provide(s) output information from the computer system (CS), e.g., a display, a speaker, one or more light-emitting diodes (LEDs), a printer, a vibration unit (e.g., for the generation of vibration alerts) and/or the like. The display may be configured to present or otherwise display information to a user, suitable examples of which include a liquid crystal display (LCD), light-emitting diode display (LED), plasma display panel (PDP) or the like.

The computer system (CS) further comprises one or more interfaces for transmitting and/or receiving information from or to other devices. Such communications interface(s) (14) may be configured to transmit and/or receive information, such as to and/or from one or more sensors, other computer(s), network(s), database(s), medication dispensing devices, or the like. The communications interface(s) (14) may be configured to transmit and/or receive information by physical (wired) and/or wireless communications links. The communications interface(s) (14) may include interface(s) to connect to a network, such as using technologies such as cellular telephone, Wi-Fi, satellite, cable, digital subscriber line (DSL), fiber optics and the like. In some examples, the communications interface(s) may include one or more short-range communications interfaces configured to connect devices using short-range communications technologies such as NFC, RFID, Bluetooth, Bluetooth LE, ZigBee, infrared (e.g., IrDA) or the like.

As indicated above, program code instructions may be stored in memory (12) and executed by processing unit (11) that is thereby programmed, to implement functions of the computer system CS, subsystems, tools and their respective elements described herein. As will be appreciated, any suitable program code instructions may be loaded onto a computer system or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

## Claims

1. A machine learning architecture for medical coding comprising:
- a serving unit, wherein the serving unit is configured to receive a reported term, to output a classification result and to receive feedback from a user,
- a classification model, wherein the classification model is configured to assign one or more medical codes to the reported term and to provide the classification result, the classification result comprising the one or more assigned codes and optionally a probability value, the probability value indicating how likely it is that the assigned code has been correctly assigned and/or that the assignment is complete,
- a data ingestion unit, wherein the data ingestion unit is configured to receive training data for training and/or re-training and/or further training of the classification model,
- a storage unit, wherein the storage unit is configured to store and provide ingested and/or processed data, model artifacts, and/or parameters,
- a transformation unit, wherein the transformation unit is configured to pre-process the training data and/or to compute model performance KPIs and/or data quality KPIs,
- a training unit, wherein the training unit is configured to train, re-train and/or further train the classification model on the basis of pre-processed training data,
- a monitoring and orchestration unit, wherein the monitoring and orchestration unit is configured to monitor the architecture and/or the classification model performance, to process data exchange and/or to reflect dependencies between units and/or to schedule (and reflect dependencies between) tasks of units.

2. The machine learning architecture according to claim 1, wherein the reported term is a textual description of a medical event.

3. The machine learning architecture according to claim 1 or 2, wherein the classification model is configured to assign the one or more medical codes to the reported terms by means of a medical dictionary.

4. The machine learning architecture according to any one of claims 1 to 3, wherein the classification model is a machine learning model that is trained on the basis of training data to assign one or more medical codes to a reported term inputted to the machine learning model.

5. The machine learning architecture according to any one of claims 1 to 4, wherein the serving unit is configured to output a plurality of possible medical codes to the user and to receive feedback from the user as to which one(s) of the codes is/are correct.

6. The machine learning architecture according to claim 5, wherein the plurality of possible medical codes is outputted to the user in the order of their probability value.

7. The machine learning architecture according to any one of claims 1 to 6, wherein the serving unit is configured to receive feedback from the user, the feedback indicating whether the one or more medical codes assigned to the reported term are correct and/or complete.

8. The machine learning architecture according to any one of claims 1 to 7, wherein the feedback is stored as user feedback data in a data storage.

9. The machine learning architecture according to any one of claims 1 to 8, wherein the data ingestion unit is configured to receive an updated dictionary and/or user feedback data and/or new training data, and wherein the transformation unit is configured to generate training pairs from the updated dictionary and/ the user feedback data and/or the new training data, wherein each training pair comprises a reference reported term and one or more associated medical codes.

10. The machine learning architecture according to claim 9, wherein the transformation unit is configured to compare the updated dictionary with a previous dictionary to identify new and/or changed entries, and to generate training pairs for the new entries and/or changed entries.

11. The machine learning architecture according to claim 9, wherein the transformation unit is configured to generate, for each incorrect or incomplete medical code assigned to a reported term, a training pair comprising the reported term and the correct or complete medical code entered and/or selected by the user.

12. The machine learning architecture according to any one of claims 1 to 11, wherein the monitoring and orchestration is configured to monitor how often the user declares a classification performed by the classification model as correct and/or incorrect and/or as complete and/or as incomplete.

13. The machine learning architecture according to any one of claims 1 to 12, wherein the monitoring and orchestration unit is configured to generate and output a notification to a user that re-training of the classification model is necessary when a pre-defined threshold number or percentage of incorrect or incomplete assignments is exceeded, or to initiate re-training of the classification model when a pre-defined threshold number or percentage of incorrect or incomplete assignments is exceeded.

14. The machine learning architecture according to any one of claims 1 to 13, wherein the machine learning architecture is provided in the form of Infrastructure-as-Code.

15. A non-transitory computer readable medium having stored thereon software instructions that, when executed by one or more processors of one or more computers, cause the computer(s) to execute the following steps:
- receiving a plurality of reported terms,
- for each received reported term:
∘ determining one or more medical codes on the basis of the reported term by means of a trained classification model, the classification model being trained on the basis of a medical dictionary to assign one or more medical codes to a reported term,
∘ outputting the one or more medical codes,
∘ receiving user feedback, the user feedback indicating whether the one or more medical codes are correct and/or complete
- monitoring the number and/or the type of incorrect and/or incomplete medical codes on the basis of the user feedback,
- initiating further training and/or re-training in case the number and/or percentage of incorrect and/or incomplete medical codes and/or incorrect and/or incomplete types of medical codes exceeds a pre-defined threshold, and/or in case an update of the medical dictionary is available.
